Europäisches Patentamt

European Patent Office

Office européen des brevets

(19)

(11) **EP 0 944 579 B1**

(12) **EUROPÄISCHE PATENTSCHRIFT**

(45) Veröffentlichungstag und Bekanntmachung des Hinweises auf die Patenterteilung:
**31.10.2001 Patentblatt 2001/44**

(51) Int Cl.$^7$: **C07C 215/08**, A61K 7/18

(21) Anmeldenummer: **97911101.0**

(86) Internationale Anmeldenummer:
**PCT/CH97/00435**

(22) Anmeldetag: **17.11.1997**

(87) Internationale Veröffentlichungsnummer:
**WO 98/22427 (28.05.1998 Gazette 1998/21)**

(54) **AMINHYDROFLUORIDE UND IHRE VERWENDUNG ALS MUNDPFLEGEMITTEL**

AMINOHYDROFLUORIDES AND THEIR USE AS ORAL HYGIENE PREPARATIONS

FLUORHYDRATES D'AMINES ET LEUR UTILISATION COMME AGENTS D'HYGIENE DENTAIRE

(84) Benannte Vertragsstaaten:
**AT BE CH DE DK ES FI FR GB GR IE IT LI LU MC NL PT SE**
Benannte Erstreckungsstaaten:
**LT LV RO SI**

(30) Priorität: **18.11.1996 CH 284396**

(43) Veröffentlichungstag der Anmeldung:
**29.09.1999 Patentblatt 1999/39**

(73) Patentinhaber: **Gaba International**
**4106 Therwil (CH)**

(72) Erfinder:
• **HECKENDORN, René**
**CH-4054 Basel (CH)**
• **GOSTELI, Jacques**
**CH-4059 Basel (CH)**

(74) Vertreter: **Zimmermann, Hans, Dr. et al**
**A. Braun Braun Héritier Eschmann AG**
**Holbeinstrasse 36-38**
**4051 Basel (CH)**

(56) Entgegenhaltungen:
**DE-B- 1 198 493          FR-A- 1 066 270**
**GB-A- 1 056 962          US-A- 3 983 175**
**US-A- 4 160 022**

EP 0 944 579 B1

**Beschreibung**

Die vorliegende Erfindung betrifft Aminhydrofluoride und Gemische dieser Aminhydrofluoride sowie ein Verfahren zur Herstellung dieser Aminhydrofluoride und deren Verwendung in Mundpflegemitteln.

Mundpflegemittel dienen bekanntlich dazu, durch ihre Reinigungswirkung einen Beitrag zur Hygiene der Mundhöhle und damit zur Gesunderhaltung von Zähnen und Zahnfleisch zu leisten. Die Reinigungswirkung dieser Mundpflegemittel wird üblicherweise durch Beimischung von Wirkstoffen ergänzt, die krankhafte Erscheinungen in der Mundhöhle, insbesondere auch die Bildung bakterieller Zahnbeläge (Plaque), verhüten oder bekämpfen. Diese Beläge bestehen aus Polysacchariden, in erster Linie aus Dextranen. Diese Polysaccharide bilden neben den niedermolekularen Zuckern eine Nahrungsquelle der Plaquebakterien (hauptsächlich Streptokokken und Lactobacillaceen). Die Plaquebakterien bauen die Polysaccharide allmählich zu sauren Abbauprodukten (z.B. Brenztraubensäure, Milchsäure usw.) ab. Die daraus resultierende pH-Absenkung bewirkt den als Karies bekannten Abbau des Zahnschmelzes.

Man hat daher schon versucht, mit verschiedenen oralen, antibakteriell wirksame Substanzen enthaltenden Mundpflegemittelmitteln (z.B. Zahnpasten, Spüllösungen oder Zahngelées) der Bildung von krankhaften Erscheinungen in der Mundhöhle entgegenzutreten. Aus dem Stand der Technik vorbekannte Wirkstoffe sind N-Octadeca-9-enylamin-hydrofluorid (internationaler Freiname "Dectaflur") und insbesondere N'-Octadecyl-N',N,N-tris(2-hydroxyethyl)-1,3-propandiamin-dihydrofluorid (internationaler Freiname "Olaflur"); beide beschrieben in der DE-OS-1 198 493. Diese Wirkstoffe bilden bei der oralen Anwendung des Mundpflegemittels auf dem Zahnschmelz einen dünnen hydrophoben Film, wobei die Aminhydrofluoridgruppen mit dem Zahnschmelz in Kontakt kommen. So wird einerseits der Zahnschmelz aufgrund der gebildeten $CaF_2$-Deckschicht resistenter gegen Säureangriffe, andererseits bilden die langkettigen Kohlenwasserstoff-Reste eine hydrophobe Schicht, die die Bildung von Ablagerungen und den Angriff der sauren Abbauprodukte auf den Zahnschmelz behindert.

Die Synthese von Olaflur geht von Rindertalg, einem Fett mit hohem Stearinsäuregehalt, aus. Die Estergruppen werden verseift, die freien Fettsäuren mit Ammoniak in die entsprechenden Amide übergeführt und diese zum Nitril dehydratisiert. Deren katalytische Reduktion liefert ein Gemisch von primären Fettaminen mit Hauptbestandteil Octadecylamin. Umsetzen mit Acrylnitril und erneute katalytische Reduktion ergibt daraus N-Octadecyl-1,3-propandiamin, das mit Ethylenoxid hydroxyethyliert wird. Hier entstehen Anteile von Nebenprodukten, da die Aminogruppen teilweise unter- oder übersubstituiert werden. Es können auch die eingeführten Hydroxyethylgruppen durch weiteres Ethylenoxid verethert werden. Die anschliessende doppelte Hydrofluoridierung liefert das Endprodukt Olaflur in technischer Reinheit, in dem N'-Octadecyl-N',N,N-tris(2-hydroxyethyl)-1,3-propandiamin-dihydrofluorid als Hauptkomponente vorkommt. Auf die Reinigung von den Nebenprodukten wird aus Kostengründen verzichtet.

Die Anwesenheit dieser Nebenprodukte wurde bislang als nicht störend erachtet, da sie in Bezug auf die Filmbildung auf dem Schmelz von untergeordneter Bedeutung sind.

Es ist jedoch damit zu rechnen, dass im Zuge der globalen Verschärfung der behördlichen Genehmigungsverfahren für pharmazeutische Wirkstoffe in Zukunft die Zulassungen für verunreinigte Wirkstoffe schwerer zu erhalten sein werden.

Der vorliegenden Erfindung liegt die Aufgabe zugrunde, Wirkstoffe bereitzustellen, die eine dem Olaflur vergleichbare Wirksamkeit aufweisen, jedoch weniger Nebenprodukte enthalten und einfacher herzustellen sind.

Die gestellte Aufgabe wird erfindungsgemäss durch Aminhydrofluoride der allgemeinen Formel (I):

$$R\text{-}N(CH_2CH_2OH)_2 \cdot HF \qquad\qquad (I)$$

gelöst, wobei R einen geradkettigen Kohlenwasserstoffrest mit 10 bis 20 Kohlenstoffatomen bedeutet.

Es wurde namlich gefunden, dass diese Aminhydrofluoride und Gemische aus zwei oder mehreren davon eine antibakterielle Wirksamkeit aufweisen, die derjenigen des N'-Octadecyl-N',N,N-tris(2-hydroxyethyl)-1,3-propandiamindihydrofluorids aus dem vorbekannten Olaflur sehr ähnlich ist. Die antibakterielle Wirkung äussert sich in der Hemmung des Wachstums einer Vielzahl von Mikroorganismen, so z.B. von Aspergillus niger, Candida albicans, Escherichia coli, Pseudomonas aeruginosa, Staphylococcus aureus, Streptococcus faecalis und Enterobacter cloacae. Die erfindungsgemässen Aminhydrofluoride hemmen insbesondere den Zuckerabbau bei säureproduzierenden Plaquebakterien und erhöhen durch die Bildung eines hydrophoben Films die Säureresistenz der Zahnhartsubstanz, haben also kariesprophylaktische Wirkung. Sie begünstigen auch die Remineralisation initialer kariöser Läsionen.

Die erfindungsgemässen Aminhydrofluoride enthalten geradkettige (d.h. unverzweigte) Kohlenwasserstoffreste. Sie können Kohlenwasserstoffreste mit sowohl gerad- wie ungeradzahliger Kettenlänge aufweisen. Im Hinblick auf die physiologische Unbedenklichkeit sind Reste mit geradzahliger Kettenlänge bevorzugt. Die Reste können vorzugsweise voll gesättigt oder einfach, doppelt oder mehrfach ungesättigt sein. Beispiele für gesättigte Kohlenwasserstoffreste mit geradzahliger Kettenlänge sind Decyl, Dodecyl (Lauryl), Tetradecyl (Myristyl), Hexadecyl (Cetyl, Palmityl), Octadecyl (Stearyl) und Eicosanyl. Beispiele für ungesättigte Reste mit geradzahliger Kettenlange sind 9-cis-Octadecenyl (Oleyl), 9-trans-Octadecenyl (Elaidyl), cis,cis-9,12-Octadecadienyl (Linolyl), cis,cis,cis-9,12,15-Octadecatrienyl (Linolenyl) oder 9-cis-Eicosaenyl (Gadolyl). Bevorzugt sind Lauryl-, Myristyl-, Cetyl-, Oleyl- und Stearylreste.

Die Aminhydrofluoride werden erfindungsgemäss hergestellt, indem man ein Amin der allgemeinen Formel (II):

$$R\text{-}N(CH_2CH_2OH)_2 \qquad\qquad (II)$$

worin R einen Kohlenwasserstoffrest mit 10 bis 20 Kohlenstoffatomen bedeutet, mit Fluorwasserstoff umsetzt. Die Umsetzung kann in allen Lösungsmitteln durchgeführt werden, die eine ausreichende Löslichkeit für das freie Amin der Formel (II) zeigen und durch Fluorwasserstoff nicht angegriffen werden. Beispiele geeigneter Lösungsmittel sind $C_1$-bis $C_4$-Alkohole und Dimethylsulfoxid; besonders bevorzugt ist Ethanol. Bevorzugt ist die Zugabe des Fluorwasserstoffs als wässrige Lösung, d.h. als Flussäure. Der Fluorwasserstoff wird vorzugsweise in einer Menge von 1 bis 5, besonders bevorzugt von 1,0 bis 1,1 Aequivalenten, bezogen auf das Amin, zugegeben. Die Temperatur der Umsetzung ist nicht kritisch und kann im allgemeinen zwischen -10 und +100°C liegen, wobei der obere Grenzwert von dem Siedepunkt des Lösungsmittels bestimmt sein kann, oder der untere Grenzwert durch den Schmelzpunkt des Lösungsmittels. Die bevorzugte Temperatur der Umsetzung liegt zwischen 25°C und 40°C. Nach der Beendigung der Zugabe des Fluorwasserstoffs kann das Reaktionsgemisch eingedampft und getrocknet werden, wodurch das erfindungsgemässe Aminhydrofluorid erhalten wird. Ein allfälliger geringer Fluorwasserstoff-Verlust während des Eindampfens und Trocknens kann durch nachträgliche Zugabe einer entsprechenden Menge Flussäure bei der Herstellung der erfindungsgemässen Formulierungen ausgeglichen werden.

Das Amin der Formel (II) kann in bekannter oder an sich bekannter Weise durch Hydroxyethylierung eines primären Amins $R\text{-}NH_2$, worin R die obige Bedeutung hat, mit Ethylenoxid (Oxiran) hergestellt werden, wobei die Ethoxylierung praktisch quantitativ nur am Stickstoffatom stattfindet.

Das primäre Amin $R\text{-}NH_2$ kann in bekannter Weise aus einer Fettsäure der Formel R-COOH, worin R die obige Bedeutung hat, mittels der Syntheseschritte Amidbildung/Dehydratisieren/katalytische Reduktion, wie sie auch in der Synthese des vorbekannten Olaflurs angewendet werden, erhalten werden. Geeignete Fettsäuren bzw. Fettsäuregemische der Formel R-COOH und geeignete Amine bzw. Amingemische dei Formel $R\text{-}NH_2$ sind bekannt und zum Teil im Handel erhältlich.

Gemäss einer weiteren Variante kann das Amin der Formel (II) durch Alkylierung von Diethanolamin in einer nukleophilen $S_N2$-Substitution hergestellt werden:

$$R\text{-}X + HN(CH_2CH_2OH)_2 \rightarrow R\text{-}N(CH_2CH_2OH)_2 \cdot HX$$

wobei R-X das Alkylierungsmittel bedeutet, R dieselbe Bedeutung wie in Formel (II) hat, und X für z.B. Chlor, Brom oder Jod steht.

Das Amin der Formel (II) wird hier zunächst als Ammoniumsalz erhalten. Dieses Ammoniumsalz wird mit Base, beispielsweise wässriger NaOH, deprotoniert und dann erfindungsgemäss mit Fluorwasserstoff umgesetzt.

Das Alkylierungsmittel R-X kann aus einem entsprechenden, im Handel erhältlichen Fettalkohol durch Einführen einer Abgangsgruppe erhalten werden:

$$R\text{-}OH + HX \rightarrow R\text{-}X + H_2O,$$

wobei R-OH den Fettalkohol bedeutet, und HX fur z.B. Chlorwasserstoff, Bromwasserstoff, Jodwasserstoff steht.

Auf der Stufe des Fettalkohols kann, z.B. durch fraktionierte Destillation und/oder Umkristallisation, eine Abtrennung von allfälligen Homologen und/oder Doppelbindungsisomeren erfolgen, so dass ein reiner Fettalkohol erhalten wird (für die erforderlichen physikalischen Daten vgl. z.B. Römpp, Chemielexikon, 9. Auflage Bd. 2, Seite 1337).

Die erfindungsgemässen Aminhydrofluoride sind vorzugsweise im wesentlichen frei von Di- oder Polyaminhydrofluoriden, wie sie für das vorbekannte Olaflur typisch sind. Sie sind, insbesondere wenn der Herstellungsweg über die Alkylierung von hochreinem Diethanolamin gewählt wird, frei von Produkten, in denen die Aminogruppen über- oder unterhydroxyethyliert sind (d.h. dass die Amine nach der Hydroxyethylierung quartär resp. sekundär sind) oder die Hydroxyethylgruppen verethert sind. Die erfindungsgemässen Aminhydrofluoride weisen, wenn der Weg über die Alkylierung von Diethanolamin gewählt wird, einen Hydroxyethylierungsgrad von genau zwei auf.

Erfindungsgemäss wird die Aufgabe auch durch ein Aminhydrofluoridgemisch, enthaltend zwei oder mehrere Verbindungen der Formel (I), gelöst.

Die erfindungsgemässen Gemische von Aminhydrofluoriden können Mischungen in beliebigem Verhältnis von zwei oder mehreren, gemäss einem der obigen Verfahren herstellbaren Aminhydrofluoriden sein.

Bevorzugt sind Gemische von Aminhydrofluoriden, die aus einem Fettsäuregemisch eines tierischen oder pflanzlichen Fettes oder Öls erhalten wurden und deren Kohlenwasserstoffreste R daher eine Häufigkeitsverteilung in Abhängigkeit von der Kettenlänge aufweisen, die die Häufigkeitsverteilung der entsprechenden Fettsäurehomologen in diesem Fettsäuregemisch widerspiegelt. Solche Gemische können durch Hydrofluoridieren von Mischungen von Aminen der Formel (II) erhalten werden. Die Mischungen von Aminen werden hier, ausgehend von einem tierischen oder pflanzlichen Fett oder Öl, unter Anwendung der Syntheseschritte Hydrolyse/Amidbildung/Dehydratisierung/katalytische Reduktion/Hydroxyethylierung in Analogie zu den entsprechenden Syntheseschritten beim vorbekannten Olaflur hergestellt.

Beispiele für die für diese Mischungen von Aminen der Formel (II) geeigneten pflanzlichen Öle oder Fette sind Mandelöl, Avocadobirnenöl, Maiskeimöl, Baumwollsamenöl, Rapssamenöl, Leinsamenöl, Olivenöl, Erdnussöl, Kürbissamenöl, Reiskleienol, Safloröl, Sesamöl, Sojaöl, Sonnenblumenöl, Weizenkeimöl, Babassufett, Kokosfett, Palmkernöl, Rapsöl und Palmfett. Beispiele für tierische Fette oder Öle sind Rindertalg, Hühnerfett, Ziegenfett, Schweineschmalz, Schaftalg, diverse Fischöle und Waltran. Ein bevorzugtes tierisches Fett ist Rindertalg; bevorzugte pflanzliche Öle sind Sojaöl, Rapsöl oder Sojaöl/Rapsöl-Mischungen. Eine ausführliche Tabelle mit den Zusammensetzungen der sich aus diesen tierischen oder pflanzlichen Fetten oder Ölen ergebenden Fettsäuregemische findet sich z.B. in Ullmann's Encyclopedia of Industrial Chemistry 5th Edition, Vol. A10, Seite 176f. Der Anteil gesättigter Fettsäuren kann, wenn gewünscht, durch katalytisches Hydrieren erhöht werden.

Gemische von Aminen der Formel (II), die aus einem pflanzlichen oder tierischen Fett oder Öl erhalten wurden, sind im Handel erhältlich. Beispiele sind die von der Firma AKZO NOBEL vertriebenen Produkte Ethomeen S/12 (erhalten aus dem Fettsäuregemisch von Sojaöl), Ethomeen T/12 und Ethomeen HT/12 (beide erhalten aus dem Fettsäuregemisch von Rindertalg, letzteres hydriert). Die Hauptkomponente bei S/12 und T/12 ist N-Oleyldiethanolamin. Die Anmelderin konnte von der Firma WITCO auch ein Gemisch von Aminen der Formel (II) beziehen, dessen Hauptkomponente N-Stearyldiethanolamin ist. Dieses Gemisch ist von dem Fettsäuregemisch des Rindertalgs erhalten, wobei die Oleylreste zu Stearylresten hydriert wurden.

Ein erfindungsgemässes Gemisch von Aminhydrofluoriden kann auch erhalten werden, wenn ein Gemisch von Aminhydrofluoriden, das aus dem Fettsäuregemisch eines pflanzlichen oder tierischen Fetts oder Öls erhalten wurde, mit einem oder mehreren reinen Aminhydrofluoriden vermischt wird. Es können auch zwei oder mehrere Gemische von Aminhydrofluoriden, die von jeweils unterschiedlichen Fetten oder Ölen erhalten wurden, vermischt sein.

Es kann auch ein Fett, wie in der Technik bekannt, zu einem Gemisch von Fettalkoholen reduziert werden und dieses Fettalkoholgemisch, wie oben für die Synthese von Aminhydrofluoriden in reiner Form aus reinen Fettalkoholen beschrieben, zu einem entsprechenden Gemisch von Aminhydrofluoriden verarbeitet werden.

Die Erfindung betrifft ebenfalls Mundpflegemittel enthaltend mindestens eines der erfindungsgemässen Aminhydrofluoride in wirksamer Menge. Sie können in Analogie zu den herkömmlichen Mundpflegemitteln und unter Verwendung der üblichen Hilfs- und Zusatzstoffe hergestellt werden.

Vorzugsweise können die erfindungsgemässen Mundpflegemittel auch ein aus einem pflanzlichen oder tierischen Öl oder Fett, insbesondere aus Rindertalg, Sojaöl, Rapsöl oder Sojaöl/Rapsöl-Mischungen erhaltenes Gemisch von Aminhydrofluoriden enthalten.

Erfindungsgemässe Mundpflegemittel können vorzugsweise neben einem Aminhydrofluorid oder einem Gemisch von Aminhydrofluoriden auch Zinn(II)fluorid enthalten. Diese zinnfluoridhaltigen Mundpflegemittel sind wirksam gegen Gingivitis, Parodontitits und Stomatitis, wobei sie durch den Gehalt an Aminhydrofluoriden ebenfalls kariesprophylaktisch wirksam sind. Das oder die erfindungsgemässen Aminhydrofluoride bewirken in solchen Mundpflegemitteln eine galenische Stabilisierung des Sn(II) gegen Praezipitation zu unlöslichem Zinn(IV)oxid.

Die untere Grenze für den Gewichtsanteil des oder der Aminhydrofluoride in dem Mundpflegemittel wird durch die noch signifikante prophylaktische, d.h. insbesondere die antimikrobielle oder kariesprophylaktische Wirkung bestimmt. Die obere Grenze für den Gewichtsanteil des oder der Aminhydrofluoride ist nicht kritisch, sie sollte jedoch nicht allzu hoch liegen im Hinblick auf mögliche toxische Nebenwirkungen.

Im Falle eines Mundpflegemittels in Form von Zahnpasten können erfindungsgemässe Aminhydrofluoride vorzugsweise in Mengen von 0,02 bis 5 Gewichtsprozenten, besonders bevorzugt 2 bis 3 Gewichtsprozenten enthalten sein.

Die Zusatz- und Hilfsstoffe für erfindungsgemässe Zahnpasten sind Scheuermittel, Bindemittel, Weichmacher, Feuchthaltemittel sowie Geschmacks- und Aromastoffe. Beispiele für Scheuermittel sind Erdalkali-Phosphate (z. B. Dicalciumphosphat-dihydrat, Dicalciumphosphat-anhydrid, Tricalciumphosphat), unlösliche Alkalimetall-metaphosphate, fein gemahlene oder kolloidale Siliciumdioxide, Aluminiumhydroxidhydrate, Aluminiumsilicate, Aluminium-Magnesium-Silicate und Erdalkalicarbonate. Es können auch geeignete Kunststoffe, z.B. Polyethylen eingesetzt werden. Diese Scheuermittel werden üblicherweise in Anteilen von 20 bis 60 Gewichtsprozenten eingesetzt. Bindemittel sind gelierende Agentien natürlichen oder synthetischen Ursprungs. Beispiele hierfür sind wasserunlösliche Alginate, Carraghenate, Guar-Gum, Tragacanth, wasserlösliche Celluloseether (z.B. Methylcellulose, Hydroxyalkylcellulosen, Carboxymethylcellulose), wasserlösliche Salze von Polyacrylsäuren (Carbopole), Aerosile und Bentonite. Im allgemeinen beträgt der Gehalt an diesen Bindemitteln 0,5 bis 10 Gewichtsprozent. Beispiele für Weichmacher und Feuchthaltemittel sind mehrwertige Alkohole wie Glycerin, Propylenglykol, Sorbit, Mannit, Glucosesirup, Polyethylenglykole, Polypropylenglykole und Polyvinylpyrrolidon. Sie werden üblicherweise in Mengen von 10 bis 40 Gewichtsprozenten eingesetzt. Beispiele für Geschmacksstoffe sind Saccharin, quaternäre Ammoniumsaccharinate, Cyclamate, Cumarin, Vanillin. Aromastoffe sind üblicherweise ätherische Öle, z.B. Pfefferminzöl, Spearmintöl, Anisöl, Menthol, Anethol, Citrusöl usw. oder sonstige Aromen wie Apfel-, Eukalyptus- oder Krauseminzaroma.

Erfindungsgemässe Spüllösungen sind vorzugsweise wässerige, alkoholische oder gemischt wässrig/alkoholische Lösungen mit einem oder mehreren der erfindungsgemässen Aminhydrofluoriden. Das oder die erfindungsgemässen Aminhydrofluoride können typischerweise in Mengen von 0,02 bis 2 Gewichtsprozenten, bevorzugt 0,2 bis 0,3 Gewichtsprozenten, enthalten sein. Zusatz- und Hilfsstoffe für Spüllösungen sind z.B. die oben erwähnten Geschmacks- und Aromastoffe, aber auch Emulgatoren, Netzmittel, Sorbit, Xylit und diverse Drogenauszüge.

Erfindungsgemässe Zahngelées enthalten als Trägermasse eine Aufquellung natürlicher oder synthetischer Hydrokolloide. Beispiele hierfür sind Methylcellulose, Hydroxyalkylcellulosen, Carboxymethylcellulose, wasserlösliche und quellbare Salze der Polyacrylsäuren, Alginate, Carraghenate und Guar-Gum. In die jeweilige Gelbasis können auch die oben erwähnten Geschmacks- und Aromastoffe und Feuchthaltemittel und eventuell auch Pigmente in kleinen Mengen beigemischt werden. Es können eines oder mehrere der erfindungsgemässen Aminhydrofluoride in Mengen von 0,02 bis 10 Gewichtsprozenten, bevorzugt 4,9 bis 5,0 Gewichtsprozenten enthalten sein. Zur Geschmackskaschierung und/oder als zusätzliche Fluoridquelle kann auch Natriumfluorid, in Mengen bis zu 5 Gewichtsprozenten, beigegeben werden.

Weitere Beispiele für erfindungsgemässe Mundpflegemittel sind Touchierlösungen und Kautabletten. Der Gehalt an Aminhydrofluoriden kann bei Touchierlösungen wahlweise höher sein als bei Spüllösungen. Er kann bei Touchierlösungen typisch 5 bis 25 Gewichtsprozente, bevorzugt 15 bis 25 Gewichtsprozente betragen. Als Zusatzstoffe können bei Touchierlösungen dieselben Substanzen Verwendung finden wie bei den Spüllösungen. Bei Kautabletten können Aminhydrofluorid-Anteile von typisch 0,3 bis 12 Gewichtsprozenten, bevorzugt 2 bis 7 Gewichtsprozenten vorhanden sein. Zusatzstoffe für Kautabletten können Bindemittel und Saccharose, Glucose, Milchzucker oder bevorzugt die nicht kariogenen Zuckerarten wie Xylit, Mannit oder Sorbit sein. Sie können durch Zugabe von Aromastoffen geschmacklich verbessert werden. Zur Herstellung von erfindungsgemässen Kautabletten können aus der herkömmlichen Herstellung von Kautabletten bekannte Verfahren und Tablettierpressen eingesetzt werden.

Bei allen erfindungsgemässen Mundpflegemitteln kann auch Zinn(II)fluorid in Mengen von in der Regel 0,001 bis 2 Gewichtsprozenten, als Feststoff oder als z.B. wässerige Lösung beigegeben werden. Es können dieselben Zusatz- und Hilfsstoffe wie bei den zinnfluoridfreien Mundpflegemitteln eingesetzt werden. Da Lösungen von Zinn(II)fluorid durch Zugabe der erfindungsgemässen Aminhydrofluoride stabilisiert werden, bleiben solche Lösungen über einen längeren Zeitraum klar und es tritt keine Trübung auf. Kombinationen der erfindungsgemässen Aminhydrofluoride und Zinn(II)fluorid eignen sich daher insbesondere für eine Anwendung in Mundflegemitteln in der Form von Spüllösungen. Spüllösungen, die wirksame Mengen von einem oder mehreren Aminhydrofluoriden und Zinn(II)fluorid enthalten, stellen eine bevorzugte Ausführungsform der erfindungsgemässen Mundpflegemittel dar.

Die vorliegende Erfindung wird nun durch die folgenden Beispiele weiter veranschaulicht. Alle Mengenangaben in Prozent, ppm und Teilen beziehen sich, sofern nicht anders erwähnt, auf Gewichte.

Beispiel 1: Herstellung von Aminhydrofluoriden

In einem geeigneten Reaktionskessel mit Rührer, Homogenisator, Temperaturregler mit Temperatur-Anzeige, Vakuum-Einrichtung sowie einer HF-beständigen Dosiervorrichtung wurde eine eingewogene Menge eines Gemisches von Aminen der Formel (II) (Ethomeen T/12 der AKZO NOBEL, typische Verteilung der Kettenlänge von R: 1 % $C_{12}$, 4 % $C_{14}$, 31 % $C_{16}$, 64 % $C_{18}$) vorgelegt, wobei diese Menge so gewählt wurde, dass die Kesselbefüllung maximal 10 bis 20 Volumenprozent betrug. Das Gemisch wurde in zwei Teilen Ethanol bei Raumtemperatur gelöst. Aus der Dosiervorrichtung wurden 1,015 Äquivalente Fluorwasserstoff in Form von 40%iger wässeriger Flussäure zugegeben. Dabei wurde darauf geachtet, dass die Temperatur des Gemisches nicht über 40°C stieg. Dann wurde die Dosiervorrichtung mit dem gleichen Volumen destilliertem Wasser nachgespült. Die Mischung wurde unter vorsichtigem Evakuieren (Schaumbildung) und einer maximalen Gemischtemperatur von 65°C zur Trockene eingedampft.

Das hier hergestellte Gemisch von Aminhydrofluoriden wird im folgenden als "Oleyl-Aminfluorid" bezeichnet.

Beispiel 2: Herstellung von Aminhydrofluoriden

Es wurde wie bei Beispiel 1 vorgegangen, ausser dass von einem anderen Gemisch von Aminen der Formel (II) (Ethomeen HT/12 der AKZO NOBEL, typische Verteilung der Kettenlänge von R: 1 % $C_{12}$, 4 % $C_{14}$, 31 % $C_{16}$, 64% $C_{18}$; durch Hydrierung im wesentlichen gesättigt) statt von Ethomeen T/12 ausgegangen wurde.

Für das hier hergestellte Gemisch von Aminhydrofluoriden wird im folgenden der Begriff "Stearyl-Aminfluorid" verwendet.

Beispiel 3: Zahnpaste

Als Reaktionsbehälter wurde ein Mischer mit Homogenisator verwendet. In den Mischer wurden 62 g Stearyl-Aminfluorid aus Beispiel 2 (entsprechend 2,48% der fertigen Zahnpaste) gegeben und bei 55°C in 1,097 kg Wasser gelöst. Danach wurden 600 g Sorbit 70%, 37,5 g Pfefferminz-Aroma, 625 g Kieselgel, 50 g Hydroxyethylcellulose (Tylose H 10 000 P, Hoechst), 25 g Titandioxid und 3,75 g Saccharin zugegeben. Das Gemisch wurde mit 100 U/min und Stufe 1 des Homogenisators während 50 Minuten bei 35°C und einem Druck von 0,5 bar, dann 15 weitere Minuten bei Raumtemperatur und einem Druck von 0,1 bar gerührt.

Beispiel 4: Zahnpaste

Als Reaktionsbehälter wurde ein Mischer mit Homogenisator verwendet. In den Mischer wurden 61,63 g Oleyl-Aminfluorid aus Beispiel 1 (entsprechend 2,465% der fertigen Zahnpaste) gegeben und bei Raumtemperatur in 0,5 kg Wasser gelost. Danach wurden 1,22 g 42,21%ige Flussäure, 600 g Sorbit 70%, 37,5 g Pfefferminz-Aroma, 596 g Wasser, 625 g Kieselgel, 50 g Hydroxyethylcellulose (Tylose H 10 000 P, Hoechst), 25 g Titandioxid und 3,75 g Saccharin zugegeben. Das Gemisch wurde mit 100 U/min und Stufe 1 des Homogenisators während 60 Minuten bei 35°C und einem Druck von 0,5 bar, dann 15 weitere Minuten bei Raumtemperatur und einem Druck von 0,1 bar gerührt.

Beispiel 5: Zahnpaste

Als Reaktionsbehälter wurde ein Mischer mit Homogenisator verwendet. In den Mischer wurden 69,5 g Stearyl-Aminfluorid aus Beispiel 2 (entsprechend 2,78% der fertigen Zahnpaste) gegeben und bei 55°C in 1,014 kg Wasser gelöst. Danach wurden 750 g Sorbit 70%, 30 g Eukalyptus-Aroma, 325 g Polyethylen, 175 g Kieselgel, 10 g Saccharin, 47,5 g Hydroxyethylcellulose (Tylose H 10 000 P, Hoechst), 25 g Titandioxid und eine Lösung von 3,75 g NaOH in 50 g Wasser zugegeben. Das Gemisch wurde mit 100 U/min und Stufe 1 des Homogenisators während 60 Minuten bei 32°C und einem Druck von 0,6 bar, dann 15 weitere Minuten bei Raumtemperatur und einem Druck von 0,1 bar gerührt.

Beispiel 6: Zahnpaste

Als Reaktionsbehälter wurde ein Mischer mit Homogenisator verwendet. In den Mischer wurden 69,0 g Oleyl-Aminfluorid aus Beispiel 1 (entsprechend 2,761% der fertigen Zahnpaste) gegeben und bei Raumtemperatur in 0,5 kg Wasser gelöst. Danach wurden 1,36 g 42,21%ige Flusssäure, 750 g Sorbit 70%, 30 g Eukalyptus-Aroma, 513 g Wasser, 325 g Polyethylen, 175 g Kieselgel, 10 g Saccharin, 47,5 g Hydroxyethylcellulose (Tylose H 10 000 P, Hoechst) und 25 g Titandioxid und eine Lösung von 3,75 g NaOH in 50 g Wasser zugegeben. Das Gemisch wurde mit 100 U/min und Stufe 1 des Homogenisators während 65 Minuten bei 38°C und einem Druck von 0,6 bar, dann 15 weitere Minuten bei 25°C und einem Druck von 0,1 bar gerührt.

Beispiel 7: Spüllösung

Die Herstellung erfolgte unter Stickstoff-Schutzgas. In einem Reaktionskessel wurden 2,48 g Stearyl-Aminfluorid aus Beispiel 2 (entsprechend 0,248% der fertigen Spüllösung) in 918 g Wasser bei 50°C gelöst. Dann wurden 2 g PEG-40-hydriertes Rizinusöl (Cremophor RH 410, BASF), 50 g Ethanol, 1 g Pfefferminz /Krauseminz-Aroma, 25 g Xylit, 250 mg Acesulfam K, 0,5 g 0,4%ige Pigmentlösung von Ariavit Blau 3.85 CI 42051 zugegeben und gelöst.

Beispiel 8: Spüllösung

Zu einer gemäss der Rezeptur von Beispiel 7 hergestellten Spüllösung wurden zusätzlich 0,575 g Zinn(II) fluorid zugegeben und gelöst.

Beispiel 9: Spüllösung

Die Herstellung erfolgte unter Stickstoff-Schutzgas. In einem Reaktionskessel wurden 25 g Xylit und 0,5 g 0,4%ige Pigmentlösung von Ariavit Blau 3.85 CI 42051 in 918 g Wasser gelöst. Dann wurden 2,47 g Oleyl-Aminfluorid aus Beispiel 1 (entsprechend 0,247% der fertigen Spüllösung), 2 g PEG-40-hydriertes Rizinusöl (Cremophor RH 410, BASF), 50 g Ethanol, 1 g Pfefferminz/Krauseminz-Aroma, 250 mg Acesulfam K und 0,048 g 42,2%ige Flussäure zugegeben und gelöst.

Beispiel 10: Spüllösung

Zu einer gemäss der Rezeptur von Beispiel 9 hergestellten Spüllösung wurden zusätzlich 0,556 g Zinn(II) fluorid zugegeben und gelöst.

Beispiel 11: Zahngelée

Als Reaktionsbehälter wurde ein Mischer mit Homogenisator verwendet. In den Mischer wurden 124 g Stearyl-Aminfluorid aus Beispiel 2 (entsprechend 4,96% des fertigen Zahngelées) gegeben und bei 90°C in 1,969 kg Wasser gelöst. Danach wurden 45 g Pfefferminz/Apfel-Aroma, 55,5 g Natriumfluorid, 10 g Saccharin, 250 g Propylenglykol und 46 g Hydroxyethylcellulose (Tylose H 10 000 P, Hoechst) zugegeben. Das Gemisch wurde mit 100 U/min und Stufe 1 des Homogenisators während 40 Minuten bei 30°C und einem Druck von 0,4 bar, dann 25 weitere Minuten bei Raumtemperatur und einem Druck von 0,1 bar gerührt.

Beispiel 12: Zahngelée

Als Reaktionsbehälter wurde ein Mischer mit Homogenisator verwendet. In den Mischer wurden 123 g Oleyl-Aminfluorid aus Beispiel 1 (entsprechend 4,93% des fertigen Zahngelées) gegeben und in 1,000 kg Wasser gelöst. Danach wurden 2,4 g 42,21%ige Flussäure, 45 g Pfefferminz/Apfel-Aroma, 55,5 g Natriumfluorid, 10 g Saccharin, 250 g Propylenglykol und 46 g Hydroxyethylcellulose (Tylose H 10 000 P, Hoechst) zugegeben. Es wurde mit weiteren 968 g Wasser verdünnt. Das Gemisch wurde mit 100 U/min und Stufe 1 des Homogenisators während 50 Minuten bei 30°C und einem Druck von 0,4 bar, dann 75 weitere Minuten bei 25°C und einem Druck von 0,1 bar gerührt.

Beispiel 13: Kautablette

15 Teile Oleyl-Aminfluorid wurden in ca. 20 Teilen Wasser suspendiert und nachfolgend durch Sprühtrocknung granuliert (Wirkstoffgranulat). Eine Mischung aus 0,6 Teilen Saccharin, 3 Teilen Plasdon (quervernetztes Polyvinylpyrrolidon), 7,5 Teilen Avicel (native Cellulose), 5 Teilen Talkum, 48 Teilen Reisstärke, 0,9 Teilen Pfefferminzöl und 220 Teilen Sorbit wurden ebenfalls durch Sprühtrocknung granuliert (Hilfsstoffgranulat). Das Wirkstoffgranulat wurde mit dem Hilfsstoffgranulat in einem Freifallmischer gemischt und auf einer handelsüblichen Exzenterpresse zu einer Charge Kautabletten tablettiert. Das Durchschnittsgewicht pro Tablette betrug 300 mg.

Beispiel 14: Touchierlösung

19,85 Teile Oleyl-Aminfluorid, entsprechend 19,85% der fertigen Touchierlösung, 0,15 Teile Saccharin, 2,5 Teilen Aromamischung (bestehend ihrerseits aus 30 Teilen Anisöl, 7,5 Teilen Menthol, 1,0 Teilen Vanillin, 6,0 Teilen Krauseminzöl und 55,5 Teilen Pfefferminzöl) sowie 77,5 Teilen Wasser wurden gemischt. Es wurde eine gebrauchsfertige Touchierlösung erhalten.

Beispiel 15: Messung des Gesamtfluoridgehalts von Aminhydrofluorid-Gemischen

Eine eingewogene Menge Oleyl-Aminfluorid von Beispiel 1 wurde in Wasser gelöst und der Fluoridgehalt durch alkalimetrische Titration mit 0,1 N wässeriger Tetrabutylammoniumhydroxid-Lösung bestimmt (Annahme äquimolarer Mengen Fluorid und Ammoniumgruppen). Die Messung ergab einen Fluoridgehalt von 4,28 %. In analoger Weise wurde für das Stearyl-Aminfluorid von Beispiel 2 ein Fluoridgehalt von 5,04 % gefunden.

Beispiel 16: Messung des Gesamtfluoridgehalts von Zahnpasten und Zahngelées

Es wurde mit einer Fluorid-Elektrode und einem Messgerät 610 der Firma METROHM gemessen. Die Kalibrierung der Elektrode erfolgte mit einer Eichlösung von 45,24 ppm Fluorid, die wie folgt zusammengesetzt war:

   a) 20 ml Fluorid-Standardlösung in Wasser (enthaltend 200 mg NaF/Liter Lösung),
   b) 20 ml TISAB-Pufferlösung pH 5,0 bis 5,5.

Der TISAB-Puffer wurde dabei wie folgt hergestellt:

Lösung I: 5 g Komplexon IV, 57 g Eisessig und 53 g Natriumchlorid in 500 g Wasser;
Lösung II: 32 g NaOH in 350 g Wasser.

Lösungen I und II wurden gemischt und mit Wasser auf 1000 ml verdünnt.

Die Messlösung der Probe wurde hergestellt, indem eine genau abgewogene Menge Probe von etwa 1 g mit Wasser auf 20 g gestellt und mit 20 g TISAB-Puffer versetzt wurde. Die Messlösung wurde unter gleichen Bedingungen wie die Eichlösung gemessen. Die Berechnung des Fluoridgehalts der Probe erfolgte mittels der allgemeinen Formel:

$$\text{ppm F}^- = 45{,}24 \cdot \frac{\text{Messwert Probelösung}}{\text{Messwert Eichlösung}} \cdot \frac{40 \text{ g}}{\text{Gewicht Probe(g)}}$$

Es wurden die in Tabelle 1 aufgeführten Gesamtgehalte gefunden.

Tabelle 1

| | |
|---|---|
| Zahnpaste aus Beispiel 3 | 1243 ppm F$^-$ |
| Zahnpaste aus Beispiel 4 | 1170 ppm F$^-$ |
| Zahnpaste aus Beispiel 5 | 1432 ppm F$^-$ |
| Zahnpaste aus Beispiel 6 | 1390 ppm F$^-$ |
| Zahngelée aus Beispiel 11 | 1,25 % F$^-$ |
| Zahngelee aus Beispiel 12 | 1,32 % F$^-$ |

Beispiel 17: Messung des Gesamtfluoridgehalts von Spüllösungen

Es wurde mit einer Fluorid-Elektrode und einem Messgerät 610 der Firma METROHM gemessen. Die Kalibrierung der Elektrode erfolgte mit einer Eichlösung von 125 ppm Fluorid, die wie folgt zusammengesetzt war:

a) 20 ml Fluorid-Standardlösung (250 ppm Fluorid) in fluoridfreier Spüllösung,
b) 20 ml TISAB-Puffer (vgl. Beispiel 16).

Die Messlösung der Probe wurde hergestellt, indem 20 ml Spüllösungs-Probe und 20 ml TISAB-Puffer gemischt wurden. Die Messlösung wurde unter gleichen Bedingungen wie die Eichlösung gemessen. Die Berechnung des Fluoridgehalts der Probe erfolgte mittels der allgemeinen Formel:

$$\text{ppm F- } = 250 \cdot \frac{\text{Messwert Probelösung}}{\text{Messwert Eichlösung}}$$

Es wurden die in Tabelle 2 aufgeführten Gesamtgehalte gefunden.

Tabelle 2

| | |
|---|---|
| aus Beispiel 9 | 250 ppm F$^-$ |
| aus Beispiel 10 | 250 ppm F$^-$ |

Beispiel 18: Bestimmung der mikrobiologischen Wirksamkeit von Aminhydrofluoriden gegen Staphylococcus aureus (ATCC 6538) und Streptococcus faecalis (ATCC 10541) im Test zur Ermittlung der minimalen Hemmstoffkonzentration (MHK-Test)

Diese beiden Mikroorganismen wurden ausgewählt, weil sie als Kokken stellvertretend für Mundhöhlenkokken von Bedeutung sind. Es wurden drei Stammlösungen aus je einem Gewichtsteil eines bakterienhaltigen Mediums, bestehend aus einer sog. Schüttelkultur auf Basis Caso-Bouillon, eingestellt auf 100000 Bakterienzellen / µl, und je einem Gewichtsteil aus drei physiologischen Kochsalzlösungen, die Oleyl-Aminfluorid aus Beispiel 1 (für Stammlösung A) bzw. Stearyl-Aminfluorid aus Beispiel 2 (für Stammlösung B) bzw. vorbekanntem Olaflur (für Stammlösung C) enthielten, hergestellt. Die Konzentration der Aminhydrofluoride in den physiologischen Kochsalzlösungen war dergestalt, dass sich in den fertigen Stammlösungen eine Fluorid-Konzentration von je 3000 ppm $F^-$ / 100 g Stammlösung ergab. Aus jeder Stammischung wurde eine geometrische Verdünnungsreihe durch Verdünnen mit physiologischer Kochsalzlösung (Verdünnungsfaktor je 2) gebildet. Es wurde untersucht, bei welchen Verdünnungen keine signifikante Hemmung der Mikroorganismen mehr beobachtet wurde. In Tabelle 3 sind die höchsten Verdunnungssstufen gegenüber den Stammischungen aufgeführt, bei denen in einem konkreten Experiment die Bakterien noch abgetötet wurden.

Tabelle 3

|  | gegenüber Stammischung A | gegenüber Stammischung B | gegenüber Stammischung C |
|---|---|---|---|
| Staph. aureus | 1 : 8192 | 1 : 2048 | 1 : 4096 |
| Str. faecalis | 1 : 8192 | 1 : 2048 | 1 : 8192 |

Unter Berücksichtigung der biologischen Variabilität in der Vitalität der Bakterien sind die drei Wirksubstanzen als in etwa gleich wirksam anzusehen.

Beispiel 19: Bestimmung der mikrobiologischen Wirksamkeit von Zahnpastenformulierungen gegen Staphylococcus aureus (ATCC 6538) und Streptococcus faecalis (ATCC.10541) im MHK-Test

Es wurden drei Stammischungen aus je einem Gewichtsteil eines bakterienhaltigen Mediums, bestehend aus einer Schüttelkultur auf Basis Caso-Bouillon, eingestellt auf 100000 Bakterienzellen / µl und einem Gewichtsteil einer Zahnpaste (Stammischung A: Zahnpaste aus Beispiel 3; Stammischung B: Zahnpaste aus Beispiel 4; Stammischung C: herkömmliche Zahnpaste mit bekanntem Olaflur) hergestellt. Der gewichtsmässige Fluoridanteil war in allen drei Stammischungen gleich. Aus jeder Stammischung wurde eine geometrische Verdünnungsreihe durch Verdünnen mit physiologischer Kochsalzlösung (Verdünnungsfaktor je 2) gebildet. Es wurde untersucht, bei welchen Verdünnungen keine signifikante Hemmung der Mikroorganismen mehr beobachtet wurde. In Tabelle 4 sind die höchsten Verdünnungssstufen gegenüber den Stammischungen aufgeführt, bei denen in einem konkreten Experiment die Bakterien noch abgetötet wurden.

Tabelle 4

|  | gegenüber Stammischung A | gegenüber Stammischung B | gegenüber Stammischung C |
|---|---|---|---|
| Staph. aureus | 1 : 512 | 1 : 4096 | 1 : 4096 |
| Str. faecalis | 1 : 256 | 1 : 4096 | 1 : 1024 |

Unter Berücksichtigung der biologischen Variabilität in der Vitalität der Mikroorganismen zeigen die beiden Zahnpasten auf Basis Oleyl-Aminfluorid bzw. Olaflur vergleichbare Wirksamkeit.

**Patentansprüche**

1.    Aminhydrofluorid der allgemeinen Formel (I):

$$R\text{-}N(CH_2CH_2OH)_2 \cdot HF \qquad (I)$$

worin R einen geradkettigen Kohlenwasserstoffrest mit 10 bis 20 Kohlenstoffatomen bedeutet.

2.    Aminhydrofluorid nach Anspruch 1, **dadurch gekennzeichnet, dass** der Kohlenwasserstoffrest R eine gerade Zahl Kohlenstoffatome aufweist.

3.    Aminhydrofluorid nach Anspruch 1 oder 2, **dadurch gekennzeichnet, dass** der Kohlenwasserstoffrest R ein Lauryl-, Myristyl-, Cetyl-, Oleyl-, Linolyl-, Linolenyl-, Stearyl-, Eicosanyl- oder Eicosaenylrest ist.

4.    Aminhydrofluorid der allgemeinen Formel (I):

$$R\text{-}N(CH_2CH_2OH)_2 \cdot HF \qquad (I)$$

worin R einen geradkettigen Kohlenwasserstoffrest mit 10 bis 20 Kohlenstoffatomen bedeutet, als antibakterielles Mittel.

**5.** Aminhydrofluorid-Gemisch enthaltend zwei oder mehrere Verbindungen der allgemeinen Formel (I):

$$R\text{-}N(CH_2CH_2OH)_2 \cdot HF \tag{I}$$

worin R einen geradkettigen Kohlenwasserstoffrest mit 10 bis 20 Kohlenstoffatomen bedeutet.

**6.** Gemisch nach Anspruch 5, erhalten aus dem Fettsäuregemisch eines pflanzlichen oder tierischen Fetts oder Öls.

**7.** Gemisch nach Anspruch 5 oder 6, erhalten aus Rindertalg, Sojaöl, Rapsöl oder einer Sojaöl/Rapsöl-Mischung.

**8.** Verfahren zur Herstellung eines Aminhydrofluorids der allgemeinen Formel (I):

$$R\text{-}N(CH_2CH_2OH)_2 \cdot HF \tag{I}$$

worin R einen geradkettigen Kohlenwasserstoffrest mit 10 bis 20 Kohlenstoffatomen bedeutet, **dadurch gekennzeichnet, dass** man ein Amin der allgemeinen Formel (II):

$$R\text{-}N(CH_2CH_2OH)_2 \tag{II}$$

worin R die obige Bedeutung hat, mit Fluorwasserstoff umsetzt.

**9.** Mundpflegemittel, umfassend mindestens ein Aminhydrofluorid der allgemeinen Formel (I):

$$R\text{-}N(CH_2CH_2OH)_2 \cdot HF \tag{I}$$

worin R einen geradkettigen Kohlenwasserstoffrest mit 10 bis 20 Kohlenstoffatomen bedeutet, in wirksamer Menge.

**10.** Mundpflegemittel nach Anspruch 9, **dadurch gekennzeichnet, dass** es ein Gemisch von aus Rindertalg, Sojaöl, Rapsöl oder einer Sojaöl/Rapsöl-Mischung erhaltenen Aminhydrofluoriden der Formel (I) umfasst.

**11.** Mundpflegemittel nach Anspruch 9 oder 10, **dadurch gekennzeichnet, dass** es in Form einer Zahnpaste vorliegt und ein oder mehrere Aminhydrofluoride der Formel (I) in einer Menge von 0,02 bis 5, vorzugsweise 2 bis 3 Gewichtsprozenten umfasst.

**12.** Mundpflegemittel nach Anspruch 9 oder 10, **dadurch gekennzeichnet, dass** es in Form einer Spüllösung vorliegt und ein oder mehrere Aminhydrofluoride der Formel (I) in einer Menge von 0,02 bis 2, vorzugsweise 0,2 bis 0,3 Gewichtsprozenten umfasst.

**13.** Mundpflegemittel nach Anspruch 9 oder 10, **dadurch gekennzeichnet, dass** es in Form eines Zahngelées vorliegt und ein oder mehrere Aminhydrofluoride der Formel (I) in einer Menge von 0,02 bis 10, vorzugsweise 4,9 bis 5,0 Gewichtsprozenten umfasst.

**14.** Mundpflegemittel nach Anspruch 9 oder 10, **dadurch gekennzeichnet, dass** es in Form einer Touchierlösung vorliegt und ein oder mehrere Aminhydrofluoride der Formel (I) in einer Menge von 5 bis 25, vorzugsweise 15 bis 25 Gewichtsprozenten umfasst.

**15.** Mundpflegemittel nach Anspruch 9 oder 10, **dadurch gekennzeichnet, dass** es in Form einer Kautablette vorliegt und ein oder mehrere Aminhydrofluoride der Formel (I) in einer Menge von 0,3 bis 12, vorzugsweise 2 bis 7 Gewichtsprozenten umfasst.

**16.** Mundpflegemittel nach einem der Ansprüche 9 bis 15, **dadurch gekennzeichnet, dass** es Zinn(II)fluorid in einer Menge von bis zu 2% umfasst.

**17.** Verwendung eines Aminhydrofluorids der allgemeinen Formel (I):

$$R\text{-}N(CH_2CH_2OH)_2 \cdot HF \tag{I}$$

worin R einen geradkettigen Kohlenwasserstoffrest mit 10 bis 20 Kohlenstoffatomen bedeutet, zur Herstellung eines Mundpflegemittels.

**18.** Verwendung eines Aminhydrofluorids nach Anspruch 17, zur Herstellung eines Mundpflegemittels mit kariesprophylaktischer Wirkung.

**19.** Verwendung eines Aminhydrofluorids nach Anspruch 17 in Kombination mit Zinn(II)fluorid, zur Herstellung eines Mundpflegemittels mit Wirkung gegen Gingivitis, Parodontitis und Stomatitis.

**Claims**

**1.** Amine hydrofluoride of the general formula (I):

$$R\text{-}N(CH_2CH_2OH)_2 \cdot HF \tag{I}$$

in which R is a straight-chain hydrocarbon residue having 10 to 20 carbon atoms.

**2.** Amine hydrofluoride according to Claim 1, **characterized in that** the hydrocarbon residue R has an even number of carbon atoms.

**3.** Amine hydrofluoride according to Claim 1 or 2, **characterized in that** the hydrocarbon residue R is a lauryl, myristyl, cetyl, oleyl, linolyl, linolenyl, stearyl, eicosanyl or eicosaenyl residue.

**4.** Amine hydrofluoride of the general formula (I):

$$R\text{-}N(CH_2CH_2OH)_2 \cdot HF \tag{I}$$

in which R is a straight-chain hydrocarbon residue having 10 to 20 carbon atoms, as an actibacterial agent.

**5.** Amine hydrofluoride mixture containing two or more compounds of the general formula (I):

$$R\text{-}N(CH_2CH_2OH)_2 \cdot HF \tag{I}$$

in which R is a straight-chain hydrocarbon residue having 10 to 20 carbon atoms.

**6.** Mixture according to Claim 5, obtained from the fatty acid mixture of a vegetable or animal fat or oil.

**7.** Mixture according to Claim 5 or 6, obtained from bovine tallow, soya oil, rape oil or a soya oil/rape oil mixture.

**8.** Process for the preparation of an amine hydrofluoride of the general formula (I):

$$R\text{-}N(CH_2CH_2OH)_2 \cdot HF \tag{I}$$

in which R is a straight-chain hydrocarbon residue having 10 to 20 carbon atoms, **characterized in that** an amine of the general formula (II):

$$R\text{-}N(CH_2CH_2OH)_2 \qquad\qquad (II)$$

in which R has the above meaning, is reacted with hydrogen fluoride.

9. Oral hygiene composition, comprising at least one amine hydrofluoride of the general formula:

$$R\text{-}N(CH_2CH_2OH)_2 \cdot HF \qquad\qquad (I)$$

in which R is a straight-chain hydrocarbon residue having 10 to 20 carbon atoms, in an efficaceous amount.

10. Oral hygiene composition according to Claim 9, **characterized in that** it comprises a mixture of amine hydrofluorides of the formula (I) obtained from bovine tallow, soya oil, rape oil or a soya oil/rape oil mixture.

11. Oral hygiene composition according to Claim 9 or 10, **characterized in that** it is present in the form of a toothpaste and comprises one or more amine hydrofluorides of the formula (I) in an amount from 0.02 to 5, preferably 2 to 3, per cent by weight.

12. Oral hygiene composition according to Claim 9 or 10, **characterized in that** it is present in the form of a rinsing solution and comprises one or more amine hydrofluorides of the formula (I) in an amount from 0.02 to 2, preferably 0.2 to 0.3, per cent by weight.

13. Oral hygiene composition according to Claim 9 or 10, **characterized in that** it is present in the form of a dental gel and comprises one or more amine hydrofluorides of the formula (I) in an amount from 0.02 to 10, preferably 4.9 to 5.0, per cent by weight.

14. Oral hygiene composition according to Claim 9 or 10, **characterized in that** it is present in the form of a topical application solution and comprises one or more amine hydrofluorides of the formula (I) in an amount from 5 to 25, preferably 15 to 25, per cent by weight.

15. Oral hygiene composition according to Claim 9 or 10, **characterized in that** it is present in the form of a chewable tablet and comprises one or more amine hydrofluorides of the formula (I) in an amount from 0.3 to 12, preferably 2 to 7, per cent by weight.

16. Oral hygiene composition according to one of Claims 9 to 15, **characterized in that** it comprises tin(II) fluoride in an amount of up to 2%.

17. Use of an amine hydrofluoride of the general formula (I):

$$R\text{-}N(CH_2CH_2OH)_2 \cdot HF \qquad\qquad (I)$$

in which R is a straight-chain hydrocarbon residue having 10 to 20 carbon atoms, for the preparation of an oral hygiene composition.

18. Use of an amine hydrofluoride according to Claim 17, for the preparation of an oral hygiene composition having caries-prophylactic action.

19. Use of an amine hydrofluoride according to Claim 17 in combination with tin(II) fluoride, for the preparation of an oral hygiene composition having action against gingivitis, parodontitis and stomatitis.

**EP 0 944 579 B1**

**Revendications**

1. Fluorhydrate d'amine de formule générale (I) :

$$R\text{-}N(CH_2CH_2OH)_2 \cdot HF \tag{I}$$

dans laquelle R représente un radical hydrocarboné à chaîne droite comportant de 10 à 20 atomes de carbone.

2. Fluorhydrate d'amine selon la revendication 1, **caractérisé en ce que** le radical hydrocarboné R comporte un nombre pair d'atomes de carbone.

3. Fluorhydrate d'amine selon la revendication 1 ou 2, **caractérisé en ce que** le radical hydrocarboné R est un radical lauryle, myristyle, cétyle, oléyle, linoléyle, linolényle, stéaryle, éicosanyle ou éicosaényle.

4. Fluorhydrate d'amine de formule générale (I):

$$R\text{-}N(CH_2CH_2OH)_2 \cdot HF \tag{I}$$

dans laquelle R représente un radical hydrocarboné à chaîne droite comportant de 10 à 20 atomes de carbone, en tant qu'agent antibactérien.

5. Mélange de fluorhydrates d'amines, contenant deux ou plusieurs composés de formule générale (I):

$$R\text{-}N(CH_2CH_2OH)_2 \cdot HF \tag{I}$$

dans laquelle R représente un radical hydrocarboné à chaîne droite comportant de 10 à 20 atomes de carbone.

6. Mélange selon la revendication 5, obtenu à partir du mélange d'acides gras d'une graisse ou d'une huile végétale ou animale.

7. Mélange selon la revendication 5 ou 6, obtenu à partir de suif, d'huile de soja, d'huile de colza ou d'un mélange d'huile de soja et d'huile de colza.

8. Procédé de préparation d'un fluorhydrate d'amine de formule générale (I) :

$$R\text{-}N(CH_2CH_2OH)_2 \cdot HF \tag{I}$$

dans laquelle R représente un radical hydrocarboné à chaîne droite comportant de 10 à 20 atomes de carbone, **caractérisé en ce qu'**on fait réagir une amine de formule générale (II):

$$R\text{-}N(CH_2CH_2OH)_2 \tag{II}$$

dans laquelle R a la signification indiquée ci-dessus, avec du fluorure d'hydrogène.

9. Produit d'hygiène buccale comprenant au moins un fluorhydrate d'amine de formule générale (I):

$$R\text{-}N(CH_2CH_2OH)_2 \cdot HF \tag{I}$$

dans laquelle R représente un radical hydrocarboné à chaîne droite comportant de 10 à 20 atomes de carbone en quantité efficace.

10. Produit d'hygiène buccale selon la revendication 9, **caractérisé en ce qu'**il comprend un mélange de fluorhydrates d'amines obtenu à partir de suif, d'huile de soja, d'huile de colza ou d'un mélange d'huile de soja et d'huile de colza.

11. Produit d'hygiène buccale selon la revendication 9 ou 10, **caractérisé en ce qu'**il se présente sous forme d'une pâte dentifrice et qu'il comprend un ou plusieurs fluorhydrates d'amines de formule (I) en une quantité de 0,02 à 5, de préférence de 2 à 3 pour-cent en poids.

12. Produit d'hygiène buccale selon la revendication 9 ou 10, **caractérisé en ce qu'**il se présente sous forme d'une eau dentaire et qu'il comprend un ou plusieurs fluorhydrates d'amines de formule (I) en une quantité de 0,02 à 2, de préférence de 0,2 à 0,3 pour-cent en poids.

13. Produit d'hygiène buccale selon la revendication 9 ou 10, **caractérisé en ce qu'**il se présente sous forme d'un gel dentaire et qu'il comprend un ou plusieurs fluorhydrates d'amines de formule (I) en une quantité de 0,02 à 10, de préférence de 4,9 à 5,0 pour-cent, en poids.

14. Produit d'hygiène buccale selon la revendication 9 ou 10, **caractérisé en ce qu'**il se présente sous forme d'une solution pour attouchement et qu'il comprend un ou plusieurs fluorhydrates d'amines de formule (I) en une quantité de 5 à 25, de préférence de 15 à 25 pour-cent en poids.

15. Produit d'hygiène buccale selon la revendication 9 ou 10, **caractérisé en ce qu'**il se présente sous forme d'une pastille à mâcher et qu'il comprend un ou plusieurs fluorhydrates d'amines de formule (I) en une quantité d 0,3 à 12, de préférence de 2 à 7 pour-cent en poids.

16. Produit d'hygiène buccale selon l'une quelconque des revendications 9 à 15, **caractérisé en ce qu'**il comprend du fluorure d'étain (II) en une quantité allant jusqu'à 2 pour-cent.

17. Utilisation d'un fluorhydrate d'amine de formule générale (I):

$$R\text{-}N(CH_2CH_2OH)_2 \cdot HF \qquad\qquad (i)$$

dans laquelle R représente un radical hydrocarboné à chaîne droite comportant de 10 à 20 atomes de carbone pour la préparation d'un produit d'hygiène buccale.

18. Utilisation d'un fluorhydrate d'amine selon la revendication 17 pour la préparation d'un produit d'hygiène buccale à effet prophylactique vis-à-vis de la carie.

19. Utilisation d'un fluorhydrate d'amine selon la revendication 17 en association avec du fluorure d'étain (II) pour la préparation d'un produit d'hygiène buccale efficace contre la gingivite, la parodontite et la stomatite.